# EUROPÄISCHE PATENTSCHRIFT

(11) **EP 3 979 929 B1**
(45) Veröffentlichungstag und Bekanntmachung des Hinweises auf die Patenterteilung: **28.02.2024**
(21) Anmeldenummer: 20731804.9
(22) Anmeldetag: 27.05.2020
(51) Int. Cl.: A61B 17/34, A61B 17/16, A61B 10/02, A61B 34/20

(54) **CHIRURGISCHES NADEL-SET ZUR POSITIONSBESTIMMUNG EINES CHIRURGISCHEN INSTRUMENTS**
SURGICAL NEEDLE SET FOR DETERMINING THE POSITION OF A SURGICAL INSTRUMENT
ENSEMBLE AIGUILLE CHIRURGICAL DE DÉTERMINATION DE LA POSITION D'UN INSTRUMENT CHIRURGICAL

(30) Priorität: 05.06.2019 DE 102019003965
(43) Veröffentlichungstag der Anmeldung: 13.04.2022
(73) Patentinhaber: Joimax GmbH, 76227 Karlsruhe (DE)
(72) Erfinder: RIES, Wolfgang, 76351 Linkenheim (DE); STEEGMÜLLER, Rainer, 70839 Gerlingen (DE)
(74) Vertreter: Lichti - Patentanwälte Partnerschaft mbB
(86) Internationale Anmeldenummer: PCT/EP2020/064716
(87) Internationale Veröffentlichungsnummer: WO 2020/245002

(56) Entgegenhaltungen:
- CA-A1- 3 071 475
- US-A1- 2010 081 920
- US-A1- 2010 174 176
- US-A1- 2013 296 691

## Beschreibung

Die Erfindung betrifft einen Obturator für ein chirurgisches Nadel-Set nach dem Oberbegriff des Anspruchs 1 und ein chirurgisches Nadel-Set mit einem solchen Obturator.

Minimal-invasive Operationen werden heute bereits mittels navigationsgestützter Operationsverfahren durchgeführt. Es werden hierzu unterschiedliche Navigationssysteme eingesetzt. Es kommen aktive und passive Systeme zum Einsatz. Bei aktiven Systemen ist ein in den Körper eines Patienten eingebrachtes Teil, wie ein Instrument oder chirurgisches Werkzeug mit einem Sender versehen, über den sich extern die Trajektorie und die Position des Instruments oder Werkzeugs, insbesondere des distalen am Eingriffsort befindlichen Endes bestimmen lässt.

Bei passiven Systemen sind neben optischen Systemen zur Positionierung eines chirurgischen Instruments, insbesondere von dessen distalem Ende auch elektromagnetische Systeme bekannt. Bei der elektromagnetischen Navigation wird durch einen Feldgenerator ein inhomogenes elektromagnetisches Feld erzeugt, welches über einen oder mehrere Sensoren erfasst wird, wodurch wiederum die Position und Ausrichtung des Instruments oder chirurgischen Werkzeugs, insbesondere dessen distales Ende, direkt oder indirekt erfasst werden kann. Direkte Erfassung des distalen Endes eines chirurgischen Teils beinhaltet die Anordnung des Sensors am distalen Ende des Teils selbst; indirekte Erfassung beinhaltet die feste starre Anbringung des Sensors in einer definierten Stelle, insbesondere Axialposition, an dem chirurgischen Teil. Aufgrund des gemessenen Sensorsignals können Rückschluss auf die Position und gegebenenfalls Ausrichtung des distalen Endes gesehen werden. Bei der passiven Navigation hat sich insbesondere die elektromagnetische Navigation bewährt, bei der ein elektromagnetisches Feld extern um den Operationsbereich, beispielsweise durch einen Erzeuger eines elektromagnetischen Feldes in einem Kissen, auf dem der Patient liegt, erzeugt wird. Im chirurgischen Instrument eingebaute spulenartige Sensoren ermöglichen die Ortung der Instrumente, woraufhin eine Darstellung in CT- oder MRT-Bildern erfolgen kann. Dieses Röntgen-Verfahren beinhaltet keine Strahlenbelastung und reduziert insgesamt so die Strahlenbelastung, auch durch einen reduzierten Röntgeneinsatz. Es wird weder die Bildqualität beeinträchtigt, noch können Sensoren, da es eben keine optischen Sensoren sind, verdeckt werden. Die Bewegungsfreiheit des Operateurs wird nicht eingeschränkt, wie es bei optischen Systemen der Fall ist.

Die Erfindung bezieht sich insbesondere auf die Ausgestaltung eines allgemein als Jamshidi-Nadel bezeichneten Hohlnadel-Sets, das erstmals von Khosrow Jamshidi in 1974 vorgeschlagen wurde (https://en.wikipedia.org/wiki/Jamshidi_needle; https://www.cancer.gov/publications/dictionaries/cancerterms/jamshidi-needle). Hierbei handelt es sich um ein Instrument aus einer Hohlnadel in Form eines Trepans und eines durch diese gesteckten Obturators, der die Hohlnadel distal in der Regel mit einer Trokarausbildung überragt. Beide Teile, Hohlnadel und Obturator, weisen proximale Griffteile auf, über die sie miteinander festlegbar sind. Während Hohlnadel und massiver Obturatorschaft aus Metall ausgebildet sind, sind die Griffteile aus Kunststoff ausgebildet. Eine solche Jamshidi-Nadel dient in der Regel zur Biopsie, insbesondere zur Knochenbiopsie, um Knochenmark aus einem Knochen, wie insbesondere aus einem Wirbelknochen der Wirbelsäule, zu entnehmen. Ein solches Nadel-Set dient darüber hinaus in der orthopädischen Chirurgie zur Punktion von Knochen, insbesondere Wirbeln, als Zugangsnadel wie zur Kyphoplastie, zur Schraubenbefestigung etc.

Es sind Systeme bekannt, bei denen an dem proximalen Ende einer solchen Jamshidi-Nadel extern ein optisches Element, sei es ein optischer Sender oder ein optischer Sensor festgelegt wird (DE 196 39 615 A1).

In beiden Fällen ist die Anbringung eines externen Teils (sei es elektromagnetischer oder optischer Sensor, sei es optischer Sender) am eigentlichen Instrument, hier insbesondere einer Jamshidi-Nadel, äußerst nachteilig, da ein solches von dem Instrument im proximalen und damit im Griffbereich radial abstehendes Element bei der Operation für den Operateur äußerst störend ist und sie ihn irritieren kann, darüber hinaus aufgrund der Festlegung durch Klemmung abfallen kann oder sich relativ zum Instrument verschieben kann, wobei im ersteren Fall der Zusammenbau während der Operation erneut erfolgen muss, im letzteren Fall die Zuordnung zur distalen Spitze, dessen Position aufgrund des Detektionssystems bei vorgegebenem Ort des Sensor- oder Senderelements bestimmt wird, nicht mehr richtig erkannt wird. Darüber hinaus kann ein solch proximal extern an einem Instrument festgelegtes Sender- oder Sensorelement zwar, wenn es fest am Instrument angeordnet ist, die Position zur Bestimmung der Position des distalen Endes des Instruments im Raum beitragen, nicht aber zur Bestimmung der Art des Instruments selbst, insbesondere vor Beginn des eigentlichen Eingriffs am Patienten durch Einführen des Instruments in den Patienten.

Die WO 2019/042579 zeigt einen Obturator, der aufwendig herzustellen ist und schwierig zu reinigen ist; darüber hinaus ist sein Zusammenwirken mit einer Hohlnadel eines chirurgischen Nadel-Sets, in die er einzustecken ist, aufgrund seiner Ausgestaltung, insbesondere im Hinblick auf den sich senkrecht zur Grifffläche des Griffteils erstreckenden Anschlussadapter nicht unproblematisch.

Der Erfindung liegt daher die Aufgabe zugrunde, einen Obturator für ein chirurgisches Nadel-Set zu schaffen, der technisch einfacher und damit im Ergebnis preiswerter herzustellen ist, eine einfache Reinigung erlaubt und insbesondere organischer mit einer Hohlnadel eines solchen Nadel-Sets zusammenwirkt.

Erfindungsgemäß wird die genannte Aufgabe durch einen gattungsgemäßen Obturator gelöst, der weiterhin die kennzeichnenden Merkmale des Anspruchs 1 aufweist.

In bevorzugter Ausgestaltung ist dabei vorgesehen, dass sich durch den Durchlass des Adapterteils bis zum distalen Ende des Hohlraums eine Sensoreinheit mit einem distalen Sensor und einem proximalen Sensor erstreckt, der letzterer durch das Adapterteil in seiner Ausrichtung einen endlichen Winkel zur Ausrichtung des ersten Sensors einschließt, wobei insbesondere der Relativwinkel zwischen erstem und zweitem Sensor zwischen 10° und 90°, vorzugsweise zwischen 50° und 70°, höchst vorzugsweise zwischen 55° und 65° liegt.

Weitere bevorzugte Ausgestaltungen der Erfindung zeichnen sich dadurch aus, dass das distale Ende des Obturators als Trokar ausgebildet ist, wobei insbesondere ein Obturatorrohr mittels eines mit ihm fest verbundenen Fixierrings an einem Griffteil des Obturators festgelegt ist und/oder dass das Adapterteil des Obturators als Luer-Adapterteil ausgebildet ist sowie darüber hinaus dass die Sensoreinheit ein mit dem Adapterteil des Obturators verbindbares Adapterteil, vorzugsweise ein komplementäres Luer-Adapterteil, aufweist.

Andere erfindungsgemäße Ausgestaltungen des Erfindungsgegenstandes sehen vor, dass Hohlnadel und Obturator durch in seitliche Ausnehmungen des Griffteils des Obturators eingreifende starre Laschen des Griffteils der Hohlnadel miteinander lösbar verbindbar sind und/oder dass die Hohlnadel als Trepan ausgebildet ist.

Darüber hinaus sieht die Erfindung in bevorzugter Ausgestaltung vor, dass die Sensoreinheit untrennbar mit einem Anschlusskabel verbunden ist, wobei insbesondere das Anschlusskabel ein Anschlussende zur Verbindung mit einer Auswerteeinheit aufweist und/oder dass die Sensoren der Sensoreinheit als Spulen ausgebildet sind, mit deren distalem und proximalem Ende jeweils eine elektrische Leitung verbunden ist. Statt einer Draht-Signalleitung ist auch eine drahtlose Übertragung einer Sensoreinheit zu einer Steuereinheit möglich.

Weitere Vorteile und Merkmale der Erfindung ergeben sich aus den Ansprüchen aus der nachfolgenden Beschreibung, in der ein Ausführungsbeispiel der Erfindung unter Bezugnahme auf die Zeichnungen im Einzelnen erläutert ist. Dabei zeigt:
- Fig. 1: Ein erfindungsgemäßes chirurgisches Nadel-Set in einer schematischen Gesamtdarstellung;
- Fig. 2: eine vergrößerte Darstellung des Details AD der Fig. 1 mit zwei miteinander verbundenen Adapterteilen eines Adapters in Form eines Luer-Adapters;
- Fig. 3: einen Schnitt entlang der Linie Z-Z in Verlängerung des Adapters der Fig. 1, 2 durch ein Griffteil eines Obturators des Nadel-Sets;
- Fig. 4: eine vergrößerte Detaildarstellung des Bereichs IV der Fig. 1 und 3;
- Fig. 5: eine Seitenansicht eines Obturators des erfindungsgemäßen Nadel-Sets;
- Fig. 6: eine Seitenansicht einer Hohlnadel des erfindungsgemäßen Nadel-Sets;
- Fig. 7: eine Draufsicht auf das Griffteil der Hohlnadel der Fig. 6;
- Fig. 8: einen Längsschnitt durch den Obturator der Fig. 5;
- Fig. 9: eine Draufsicht auf das proximale Ende des Obturators der Fig. 5, 8;
- Fig. 10: einen Längsschnitt durch das Nadelset mit durch die Hohlnadel eingestecktem Obturator;
- Fig. 11: einen Längsschnitt durch das erfindungsgemäße chirurgische Nadel-Sets gemäß der Fig. 1;
- Fig. 12: eine Draufsicht auf die proximale Stirnseite des Nadel-Sets der Fig. 11;
- Fig. 13: eine vergrößerte Darstellung des distalen Bereichs YQ der Fig. 11 mit in den Obturator eingesteckter Sensoreinheit mit einer ersten Ausgestaltung eines proximalen Sensors,
- Fig. 14: eine vergrößerte Darstellung des Bereichs X der Fig. 11 mit proximalem Sensor und
- Fig. 15: eine vergrößerte Darstellung einer alternativen Ausgestaltung eines Adapters und den dort angeordneten proximalen Sensor.

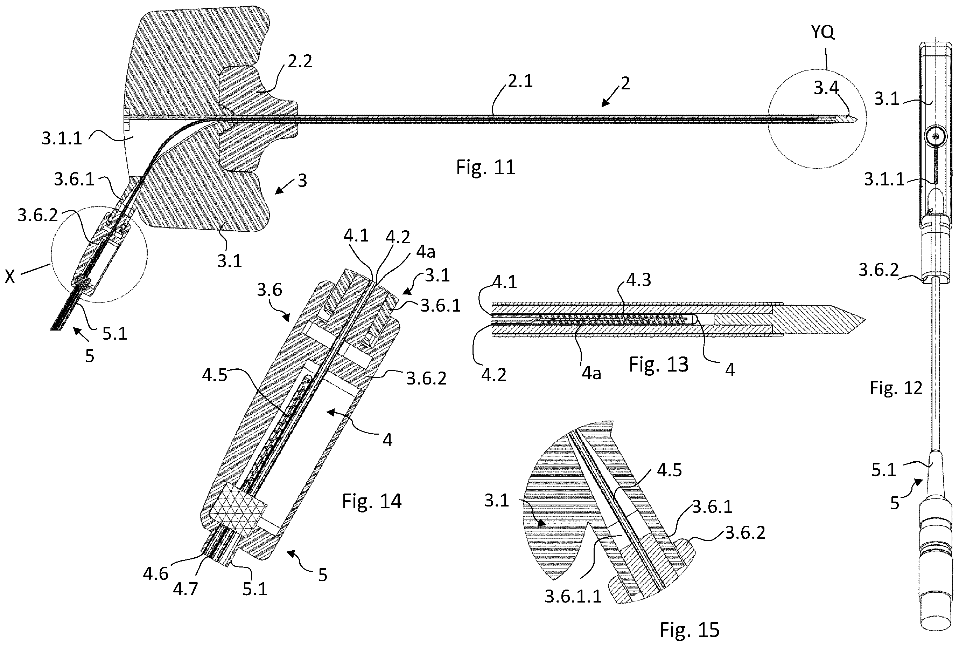

Das erfindungsgemäße chirurgische Nadel-Set 1 weist im Wesentlichen eine Hohlnadel 2, einen Obturator 3 und eine Sensoreinheit 4 (Fig. 11) auf.

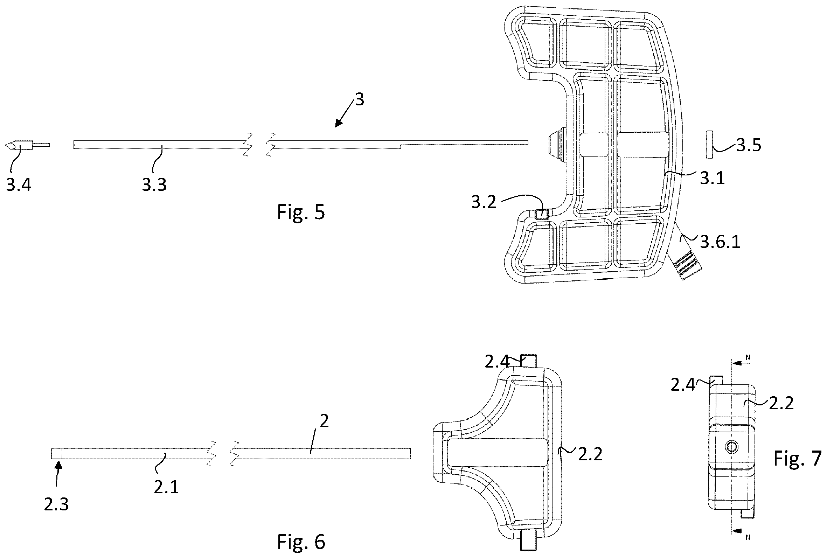

Die Hohlnadel 2 (Fig. 6) ist als Trepan ausgebildet und weist ein Hohlnadelrohr 2.1, vorzugsweise aus Edelstahl, sowie ein mit diesem am proximalen Ende verbundenes Griffteil 2.2, vorzugsweise aus Kunststoff, wie Polypropylen auf. Die beiden Teile 2.1 und 2.2 sind untrennbar verbunden, d.h. sie können nicht ohne Zerstörung getrennt werden. Das Hohlnadelrohr 2.1 ist an seinem distalen Ende 2.3 mit einer scharfen stirnseitigen kreisförmigen umlaufenden Kante versehen. Hierzu läuft die äußere Wandseite des Hohlnadelrohrs schräg nach innen auf den Radius der Innenwandseite zu.

Das Griffteil 2.2 ist T-förmig ausgebildet und weist an seinen seitlichen Enden zwei starre Laschen 2.4 auf, die mit seitlichen Ausnehmungen 3.2 eines Griffteils 3.1 des Obturators 3 durch Einschieben des Obturators 3 in die Hohlnadel 2 und relatives Verschwenken der Teile 2 und 3 zur Festlegung beider Teile 2 und 3 miteinander in Eingriff gelangen.

Der Obturator 3 (Fig. 5) weist ein hohles Obturatorrohr 3.3, das schon genannte Griffteil 3.1 sowie eine massive distale Spitze und einen Fixierring 3.5 auf.

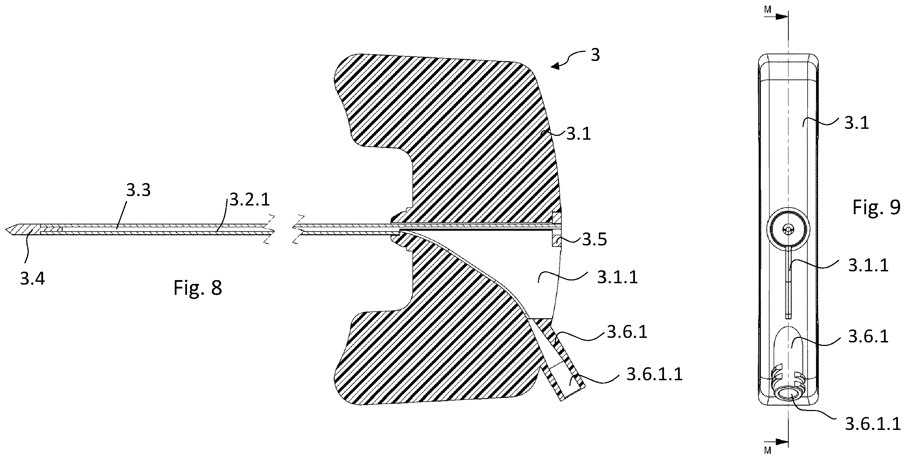

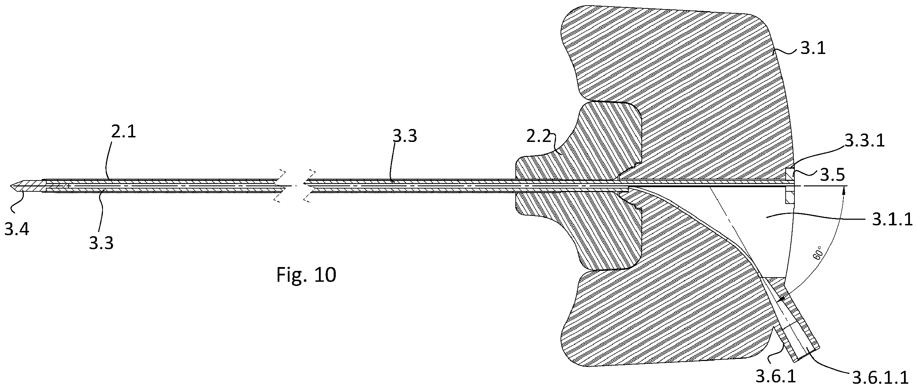

Die Teile 3.3 bis 3.5 bestehen aus Metall, vorzugsweise ebenfalls aus Edelstahl und sind fest miteinander verbunden, vorzugsweise durch Verschweißen. Die Spitze 3.4 ist als scharfer, spitzer Trokar ausgebildet. Der Haltering 3.5 ist am proximalen Ende des Obturatorrohrs 3.3 mit diesem fest und unlösbar, wie eben durch das genannte Schweißen verbunden, nachdem das Obturatorrohr 3.3 von der distalen Seite durch das Griffteil 3.1 des Obturators 3 hindurchgesteckt wurde, bis es in eine proximale Vertiefung des Griffteils 3.1 reicht, in die der Befestigungsring 3.5 zur Verbindung mit dem Obturatorrohr 3.3 eingesetzt ist (Fig. 9, 10). Das Obturatorrohr 3.3 kann auch zusätzlich oder alternativ als Einlegeteil im Griffteil 3.1 im proximalen Bereich eingespritzt sein.

Das Obturatorrohr 3.3 des Obturators 3 ist in seinem im Griffteil 3.1 liegenden Bereich nur als Halbrohr mit einer sich lediglich über die Hälfte des Umfangs erstreckenden Mantelwandung 3.3.1 ausgebildet - während es über seinen zum Griffteil 3.1 herausragenden Hauptbereich als vollumfänglich geschlossenes Rohr ausgebildet ist. Das Griffteil 3.1 weist gegenüber der Öffnung des Teilrohres 3.3.1 einen Schlitz 3.1.1 auf, der bis zu einem einstückig mit dem Griffteil ausgebildeten männlichen Adapterteil 3.6.1 wie eines Luer-Adapters reicht, dessen Durchlass 3.6.1.1 in den Schlitz 3.1.1 mündet. Derart ist eine durchgehende Hohlraumverbindung zwischen Adapterteil 3.6.1 über den Schlitz 3.1.1 zum Inneren des Obturatorrohrs 3.3 (insbesondere Fig. 10) gegeben.

Mit dem männlichen Adapterteil 3.6.1 des Adapters 3.6, das ein äußeres Schraubgewinde aufweist, ist ein weibliches Adapterteil 3.6.2 des Adapters 3.6 verbindbar, das ein inneres Gewinde aufweist (Fig. 11, 14). Das Adapterteil 3.6.2 weist weiterhin Kontaktleitungen einer Sensoreinheit 4 auf.

Die Sensoreinheit 4 weist distal des Adapters 3.6 zwei isolierte elektrische Leiter 4.1, 4.2 (Fig. 13, 14) in einem Schlauch 4a auf, die an ihren Enden mit einem distalen Sensor 4.3 in Form einer elektromagnetischen Spule verbunden sind und zwar der Leiter 4.1 mit dem proximalen Ende des Sensors 4.3, während der Leiter 4.2 durch das Innere des spulenförmigen Sensors 4.3 bis zu dessen distalen Ende hindurchgeführt und dort mit dem distalen Ende des Sensors 4.3 verbunden ist. Diese elektrisch leitenden Teile der Sensoreinheit 4 sind durch einen isolierenden Mantel ummantelt. Proximal weist die Sensoreinheit 4 einen weiteren proximalen Sensor 4.5 auf, der in gleicher Weise ausgebildet ist wie der Sensor 4.3 (Fig. 14). Bei vollständig durch das männliche Adapterteil 3.6.1 in den Obturator eingesteckter Sensoreinheit 4, bei der der distale Sensor 4.3 im Inneren des Obturatorrohres 3.3 nahe der Spitze 3.4 des Obturators zu liegen kommt, befindet sich der proximale Sensor 4.5 im abgewinkelten männlichen Adapterteil 3.6.1 des Adapters 3.6.

Die Sensoreinheit 4 weist weiterhin in der in Fig. 14 dargestellten Ausgestaltung ein weibliches Adapterteil 3.6.2 und einen proximalen Sensor 4.5 auf, der ebenfalls als Spule ausgebildet und mit zwei Leiter 4.6, 4.7 verbunden ist.

Bei der Ausgestaltung der Fig. 14 ist der proximale Sensor 4.5 distal des Adapterteils 3.6.2 im Schlauch 4a des Sensors innerhalb und in Verbindung der Adapterteile 3.6.1, 3.6.2 des Adapterteils 3.6 angeordnet.

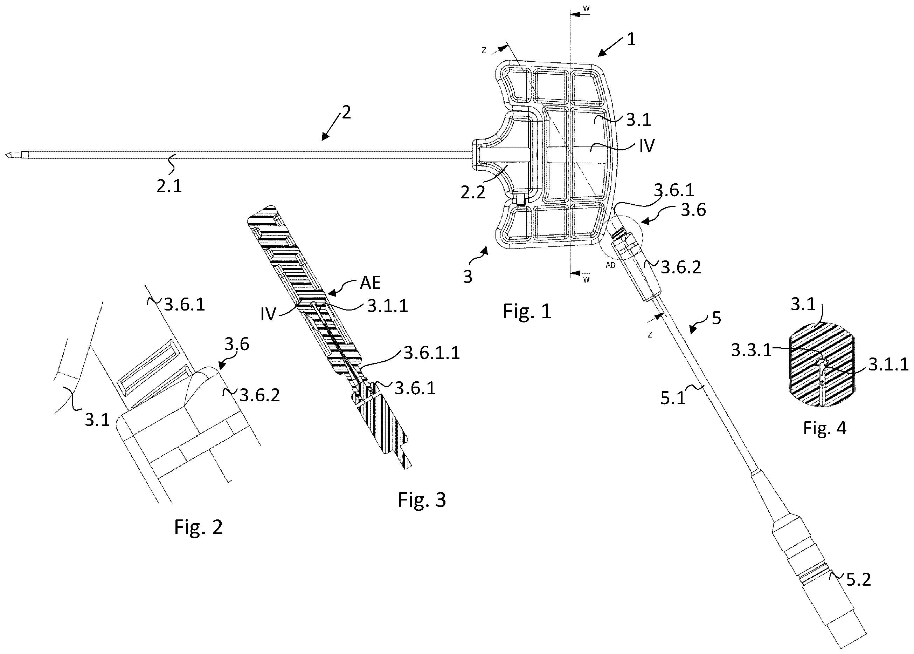

Die Sensoreinheit 4 ist fest mit einem Kabel 5 verbunden; seine Leiter 4.1, 4.2, 4.6, 4.7 führen durch das Kabel 5 bis zu dessen proximalen Anschlussende 5.2 (Fig. 1), durch das das Kabel 5 mit einem elektronischen Auswertungsgerät lösbar verbunden werden kann.

Die Adapterteile 3.6.1, 3.6.2 haben eine Ausrichtung zur Achse des Obturatorrohres 3.3 mit einem Winkel α von 60° (wie dies in Fig. 10 dargestellt ist). Der Winkel α muss ungleich 0 sein, kann aber ansonsten weitgehend beliebig sein, er muss sich nur von entsprechenden Ausrichtungen von Sensoren 4.3, 4.5 festlegenden Kavitäten anderer chirurgischer Instrumente unterscheiden, damit durch Detektion der Winkelausrichtung zwischen dem Sensor 4.3 und dem Sensor 4.5 bei Einstecken in einem Instrument das entsprechende chirurgische Instrument erkannt und im vorliegenden Falle insbesondere die hier gegebene Jamshidi-Nadel erkannt werden kann. Insofern kann der Winkel auch einen festen Wert zwischen 55° und 65° oder auch zwischen beispielsweise 40° und 80° einschließen.

Durch die Erfindung kann vorab das eingesetzte chirurgische Instrument, hier eine Jamshidi-Nadel, detektiert werden, indem vom Generator eines elektromagnetischen Feldes ein elektrisches Feld erzeugt wird und eine Auswerteeinheit nicht nur die unterschiedliche Position der beiden Sensorspulen 4.3, 4.5 auch ihr Längsabstand, sondern darüber hinaus ihre durch das entsprechende chirurgische Instrument (hier Jamshidi-Nadel) gegebene Winkelausrichtung über die durch die Sensoren 4.3, 4.5 erfassten Feldsignale bestimmt wird.

Weiterhin bietet die Erfindung in üblicher Weise die Bestimmung des Ortes des distalen Endes des chirurgischen Instruments 1 im Patienten während der Operation und aufgrund der unterschiedlichen Ausrichtung der Sensoren 4.3, 4.5 darüber hinaus auch die Ausrichtung des Instruments selbst im Raum.

Hierzu wird vorab, d.h. vor Vornahme eines Operationsschritts die Sensoreinheit 4 in das chirurgische Instrument 1, hier genauer den Obturator 3 des chirurgischen Instruments über das Adapterteil 3.6.1 eingeführt sowohl die Sensoreinheit 3 als auch das zur nicht dargestellten Auswerteeinheit führende Kabel 5 mittels des weiblichen Adapterteils 3.6.2 des Adapters 3.6 am männlichen Adapterteil 3.6.1 festgelegt, wodurch die Position der Sensoreinheit 3 und damit die der Sensoren 4.3, 4.5 im chirurgischen Instrument 1 und insbesondere im Obturator 3 exakt festgelegt ist und darüber hinaus auch durch die Positionierung der einen fest vorgegebenen Abstand an der Sensoreinheit aufweisenden Sensoren 4.3, 4.5 deren Relativausrichtung.

## Patentansprüche

1. Obturator für ein chirurgisches Nadel-Set, der beginnend mit endlichem Abstand zu seiner distalen Spitze (3.4) einen sich in proximaler Richtung längs erstreckenden Hohlraum aufweist, wobei innerhalb eines Griffteils des Obturators (3) zumindest ein proximaler, seitlicher Auslass des Hohlraums vorgesehen ist und der Hohlraum mit einem sich unter einem endlichen Winkel zu seiner Längserstreckung erstreckenden Durchlass (3.6.1.1) eines Adapterteils (3.6.1) eines Adapters in Verbindung steht,
**dadurch gekennzeichnet,**
- **dass** ein Obturatorrohr (3.3) des Obturators (3) in seinem im Griffteil (3.1) liegenden Bereich nur als Halbrohr mit einer sich lediglich über die Hälfte des Umfangs erstreckenden Mantelwandung (3.3.1) ausgebildet ist und
- **dass** das Griffteil (3.1) gegenüber der Öffnung des Teilrohres (3.3.1) einen Schlitz (3.1.1) aufweist, der bis zu einem einstückig mit dem Griffteil (3.1) ausgebildeten männlichen Adapterteil (3.6.1) reicht, dessen Durchlass (3.6.1.1) in den Schlitz (3.1.1) mündet.

2. Obturator nach Anspruch 1, **dadurch gekennzeichnet,**
**dass** sich durch den Durchlass (3.6.1.1) des Adapterteils (3.6.1) bis zum distalen Ende des Hohlraums eine Sensoreinheit (4) mit einem distalen Sensor (4.3) und einem proximalen Sensor (4.5) erstreckt, der letzterer durch das Adapterteil (3.6.1) in seiner Ausrichtung einen endlichen Winkel zur Ausrichtung des ersten Sensors (4.3) einschließt.

3. Obturator nach Anspruch 2, **dadurch gekennzeichnet,**
**dass** der Relativwinkel zwischen erstem und zweitem Sensor (4.3, 4.5) zwischen 10° und 90°, vorzugsweise zwischen 50° und 70°, höchst vorzugsweise zwischen 55° und 65° liegt.

4. Obturator nach einem der vorangehenden Ansprüche,
**dadurch gekennzeichnet, dass** sein distales Ende als Trokar ausgebildet ist.

5. Obturator nach einem der vorangehenden Ansprüche,
**dadurch gekennzeichnet, dass** ein Obturatorrohr (3.3) mittels eines mit ihm fest verbundenen Fixierrings (3.5) an einem Griffteil (3.1) des Obturators (3) festgelegt ist.

6. Obturator nach einem der vorangehenden Ansprüche,
**dadurch gekennzeichnet, dass** das Adapterteil (3.6.1) als Luer-Adapterteil ausgebildet ist.

7. Obturator nach einem der Ansprüche 2 bis 6, **dadurch gekennzeichnet, dass** die Sensoreinheit (4) untrennbar mit einem Anschlusskabel (5) verbunden ist.

8. Obturator nach Anspruch 7, **dadurch gekennzeichnet,**
**dass** dass das Anschlusskabel ein Anschlussende (5.2) zur Verbindung mit einer Auswerteeinheit aufweist.

9. Obturator nach einem der Ansprüche 2 bis 8, **dadurch gekennzeichnet, dass** Sensoren (4.3 bzw. 4.5) der Sensoreinheit als Spulen ausgebildet sind, mit deren distalem und proximalem Ende jeweils eine elektrische Leitung (4.1, 4.2 bzw. 4.8, 4.9) verbunden ist.

10. Chirurgisches Nadel-Set mit einer Hohlnadel und mit einem Obturator nach einem der Ansprüche 1 bis 9.

11. Set nach Anspruch 10, **dadurch gekennzeichnet, dass** Hohlnadel (2) und Obturator (3) durch in seitliche Ausnehmungen (3.2) des Griffteils (3.1) des Obturators (3) eingreifende starre Laschen (2.4) des Griffteils (2.2) der Hohlnadel (2) miteinander lösbar verbindbar sind.

12. Set nach Anspruch 10 oder 11 **dadurch gekennzeichnet,**
**dass** die Hohlnadel (2) als Trepan ausgebildet ist.

13. Set nach einem der Ansprüche 10 bis 12, **dadurch gekennzeichnet, dass** die Sensoreinheit (4) in einem weiblichen Adapterteil (3.6.2) ausgebildet ist.

## Claims

1. Obturator for a surgical needle set, which has, starting a finite distance from its distal tip (3.4), a hollow space extending longitudinally in a proximal direction, wherein at least one proximal lateral outlet of the hollow space is provided within a handle part of the obturator (3), and the hollow space is in connection with a passage (3.6.1.1) of an adapter part (3.6.1) of an adapter, which passage extends at a finite angle to the longitudinal extent of the hollow space,
**characterized in**
- **that** an obturator tube (3.3.) of the obturator (3) in a region located in the handle part (3.1) is in the form of merely a half-tube with a jacket wall (3.3.1) extending over only half the circumference and in
- **that** the handle part (3.1) has a slot (3.1.1) which extends to a male adapter part (3.6.1) formed in one piece with the handle part (3.1), the passage of which (3.6.1.1) opens into the slot (3.1.1).

2. Obturator according to claim 1, **characterized in that** a sensor unit (4) comprising a distal sensor (4.3) and a proximal sensor (4.3) extends through the passage (3.6.1.1) of the adapter part (3.6.1) to the distal end of the hollow space, the orientation of said proximal sensor (4.3) enclosing a finite angle to the orientation of the first sensor (4.3) due to the adapter part (3.6.1).

3. Obturator according to claim 2, **characterized in that** the relative angle between the first and the second sensor (4.3, 4.5) is between 10° and 90°, preferably between 50° and 70°, most preferably between 55° and 65°.

4. Obturator according to any of the preceding claims, **characterized in that** the distal end of the obturator is in the form of a trocar.

5. Obturator according to any of the preceding claims, **characterized in that** an obturator tube (3.3) is secured to a handle part (3.1) of the obturator (3) by means of a fixing ring (3.5) fixedly connected to said tube.

6. Obturator according to any of the preceding claims, **characterized in that** the adapter part (3.6.1) is in the form of a Luer adapter part.

7. Obturator according to any of claims 2 to 6, **characterized in that** the sensor unit (4) is inseparably connected to a connection cable (5).

8. Obturator according to claim 7, **characterized in that** the connection cable has a connection end (5.2) for connection to an evaluation unit.

9. Obturator according to any of claims 2 to 8, **characterized in that** sensors (4.3 and 4.5) of the sensor unit are in the form of coils, to the distal and proximal ends of each of which an electrical line (4.1, 4.2 and 4.8, 4.9, respectively) is connected.

10. Surgical needle set comprising a hollow needle and an obturator according to any of claims 1 to 9.

11. Set according to claim 10, **characterized in that** the hollow needle (2) and the obturator (3) are detachably connectable by means of rigid tabs (2.4) of the handle part (2.2) of the hollow needle (2), which tabs engage in lateral recesses (3.2) of the handle part (3.1) of the obturator (3).

12. Set according to claims 10 or 11, **characterized in that** the hollow needle (2) is in the form of a trephine.

13. Set according to any of claims 10 to 12, **characterized in that** the sensor unit (4) is formed in the female adapter part (3.6.2).

## Revendications

1. Obturateur pour un kit d'aiguilles chirurgicales, lequel obturateur présente une cavité s'étendant longitudinalement dans le sens proximal, en commençant à une distance finie de sa pointe (3.4) distale, dans lequel au moins une sortie proximale et latérale de la cavité est prévue à l'intérieur d'une partie de préhension de l'obturateur (3) et la cavité est en liaison avec un passage (3.6.1.1) d'une partie d'adaptateur (3.6.1) d'un adaptateur, lequel passage s'étend selon un angle fini par rapport à son extension longitudinale,
**caractérisé en ce**
- **qu'**un tube d'obturateur (3.3) de l'obturateur (3), dans sa région située dans la partie de préhension (3.1), est réalisé uniquement sous forme de demitube comportant une paroi enveloppante (3.3.1) qui ne s'étend que sur la moitié de la circonférence et
- **que** la partie de préhension (3.1) présente, opposée à l'ouverture du tube partiel (3.3.1), une fente (3.1.1), qui s'étend jusqu'à une partie d'adaptateur (3.6.1) mâle réalisée d'un seul tenant avec la partie de préhension (3.1), dont le passage (3.6.1.1) débouche dans la fente (3.1.1).

2. Obturateur selon la revendication 1, **caractérisé en ce qu'**une unité de capteur (4) comportant un capteur distal (4.3) et un capteur proximal (4.5) s'étend à travers le passage (3.6.1.1) de la partie d'adaptateur (3.6.1) jusqu'à l'extrémité distale de la cavité, ledit capteur proximal formant, à travers la partie d'adaptateur (3.6.1) dans son orientation, un angle fini par rapport à l'orientation du premier capteur (4.3).

3. Obturateur selon la revendication 2, **caractérisé en ce que** l'angle relatif entre les premier et second capteurs (4.3, 4.5) est compris entre 10° et 90°, de préférence entre 50° et 70°, de manière hautement préférée entre 55° et 65°.

4. Obturateur selon l'une des revendications précédentes,
**caractérisé en ce que** son extrémité distale est réalisée sous forme d'un trocart.

5. Obturateur selon l'une des revendications précédentes,
**caractérisé en ce qu'**un tube d'obturateur (3.3) est fixé à la partie de préhension (3.1) de l'obturateur (3) à l'aide d'un anneau de fixation (3.5) solidaire du tube d'obturateur.

6. Obturateur selon l'une des revendications précédentes,
**caractérisé en ce que** la partie d'adaptateur (3.6.1) est réalisée sous forme d'une partie d'adaptateur de Luer.

7. Obturateur selon l'une des revendications 2 à 6, **caractérisé en ce que** l'unité de capteur (4) est reliée de manière indissociable à un câble de raccordement (5).

8. Obturateur selon la revendication 7, **caractérisé en ce que** le câble de raccordement présente une extrémité de raccordement (5.2) destinée à être reliée à une unité d'évaluation.

9. Obturateur selon l'une des revendications 2 à 8, **caractérisé en ce que** les capteurs (4.3 et 4.5) de l'unité de capteur sont réalisés sous forme de bobines aux extrémités distale et proximale desquelles respectivement une ligne électrique (4.1, 4.2 et 4.8, 4.9) est reliée.

10. Kit d'aiguilles chirurgicales comportant une aiguille creuse et un obturateur selon l'une des revendications 1 à 9.

11. Kit selon la revendication 10, **caractérisé en ce que** l'aiguille creuse (2) et l'obturateur (3) peuvent être reliés de façon amovible par des pattes (2.4) rigides de la partie de préhension (2.2) de l'aiguille creuse (2), lesquelles pattes viennent en prise dans des évidements (3.2) latéraux de la partie de préhension (3.1) de l'obturateur (3).

12. Kit selon la revendication 10 ou 11, **caractérisé en ce que** l'aiguille creuse (2) est réalisée sous la forme d'un trépan.

13. Kit selon l'une des revendications 10 à 12, **caractérisé en ce que** l'unité de capteur (4) est réalisée dans une partie d'adaptateur femelle (3.6.2).
